(19) Europäisches Patentamt
European Patent Office
Office européen des brevets

(11) Publication number: **0 023 103**
**A1**

(12) # EUROPEAN PATENT APPLICATION

(21) Application number: 80302237.5

(22) Date of filing: 02.07.80

(51) Int. Cl.$^3$: **C 07 D 233/60**
C 07 D 405/06, A 61 K 31/415

(30) Priority: 04.07.79 GB 7923372

(43) Date of publication of application:
28.01.81 Bulletin 81/4

(84) Designated Contracting States:
AT BE CH DE FR GB IT LI LU NL SE

(71) Applicant: Pfizer Limited
Ramsgate Road
Sandwich Kent CT13 9NJ(GB)

(84) Designated Contracting States:
GB

(71) Applicant: Pfizer Corporation
Calle 15 1/2 Avenida Santa Isabel
Colon(PA)

(84) Designated Contracting States:
BE CH DE FR IT LI LU NL SE AT

(72) Inventor: Gymer, Geoffrey Edward
19 Palmer Road
Wingham, Canterbury Kent(GB)

(72) Inventor: Richardson, Kenneth
48 St.Stephens Hill
Canterbury, Kent(GB)

(74) Representative: Moore, James William
Pfizer Limited Ramsgate Road
Sandwich Kent CT13 9NJ(GB)

(54) **Antifungal imidazole derivatives, process for their preparation and pharmaceutical compositions thereof.**

(57) 2-Aryl-2-hydroxy-1-(1-imidazolyl)-alkanes and alkenes of the formula:

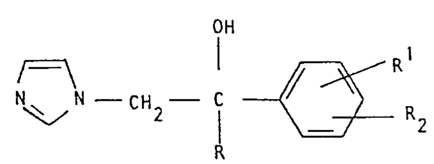

where R is a straight or branched chain alkyl or alkenyl group of from 2 to 10 carbon atoms or a cycloalkylalkyl group of from 4 to 10 carbon atoms and $R^1$ and $R^2$ are each hydrogen, halogen, lower alkyl or lower alkoxy and pharmaceutically acceptable salts thereof are antifungal agents useful in the treatment of fungal infections in animals including humans. Processes for their preparation and pharmaceutical compositions are described.

EP 0 023 103 A1

This invention relates to novel imidazole derivatives and in particular to 2-aryl-2-hydroxy-1-(1-imidazolyl)-alkanes and alkenes which have antifungal activity and are useful in the treatment of fungal infections in animals, including humans.

Thus according to the invention there are provided compounds of the formula:

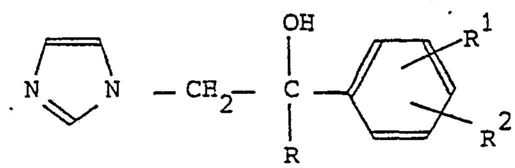

--- (I)

wherein R is a straight or branched chain alkyl or alkenyl group of from 2 to 10 carbon atoms, or a cycloalkylalkyl group of from 4 to 10 carbon atoms in which the cycloalkyl moiety contains 3 to 6 carbon atoms;

$R^1$ and $R^2$ are each independently a hydrogen or halogen atom, or a lower alkyl or lower alkoxy group;

and pharmaceutically acceptable salts thereof.

The terms "lower alkyl" and "lower alkoxy" as used herein refer to straight and branched chain groups having from 1 to 4 carbon atoms. Halogen means fluorine, chlorine, bromine or iodine. When R is a cycloalkylalkyl group, the cycloalkyl group is preferably a cyclopropyl group. $R^1$ and $R^2$ are preferably chlorine atoms, particularly in the 2- and 4- positions. R is preferably a $C_3$-$C_6$ straight or branched chain alkyl group or a $C_3$-$C_4$ alkenyl group; particularly preferred are propyl, n-butyl, isobutyl, n-hexyl and allyl groups.

BAD ORIGINAL

Particularly preferred individual compounds of the invention include:

2-(2,4-dichlorophenyl)-1-(1-imidazolyl)-pentan-2-ol

2-(2,4-dichlorophenyl)-1-(1-imidazolyl)-hexan-2-ol

2-(2,4-dichlorophenyl)-1-(1-imidazolyl)-4-methyl-pentan-2-ol

2-(2,4-dichlorophenyl)-1-(1-imidazolyl)-octan-2-ol

and

2-(2,4-dichlorophenyl)-1-(1-imidazolyl)-pent-4-en-2-ol.

The invention also provides a pharmaceutical composition comprising a compound of the formula (I) or a pharmaceutically acceptable salt thereof together with a pharmaceutically acceptable diluent or carrier.

The invention further provides a compound of the formula (I) or a pharmaceutically acceptable salt or pharmaceutical composition thereof for use in treating fungal infections in animals including humans.

The compounds of the formula (I) may be obtained by a number of different processes. In one process according to the invention they are obtained by reaction of a ketone of the formula:

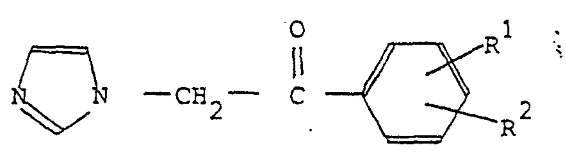

--- (II)

where $R^1$ and $R^2$ are as previously defined, with an appropriate Grignard reagent of the formula R Mg X where X is chloro, bromo or iodo and R is as previously defined.

The Grignard reagents may be prepared in an entirely conventional and well known manner by reaction of magnesium, usually in the form of magnesium turnings, with the appropriate alkyl or alkenyl chloride, bromide or iodide, in a reaction-inert organic solvent, e.g. diethyl ether. The chlorides are generally preferred because of the better yields. The reaction with the ketone (II) is again performed in a conventional manner, for example, by addition of a solution of the ketone in an inert organic solvent, e.g. tetrahydrofuran, diethyl ether or toluene, or mixtures thereof, to the freshly prepared Grignard reagent. The reaction is allowed to proceed at room temperature for some hours (generally overnight) or at reflux temperature, depending on the reactivity of the Grignard reagent. The product is then recovered in a conventional manner, e.g. by adding aqueous ammonium chloride, separating the organic phase and evaporating the solvent. The crude product may be further purified if necessary e.g. by recrystallisation or by column chromatography.

In an alternative process according to a further aspect of the invention the compounds of formula (I) other than those where R is branched or unsaturated on its $\alpha$-carbon atom are prepared from a compound of the formula (II) by first reacting with trimethyloxosulphonium methylide to give the oxirane (III):

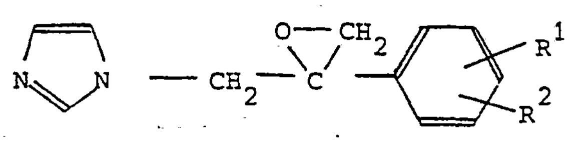

--- (III)

The oxirane is then reacted with a Grignard reagent as before but naturally choosing a Grignard reagent containing one methylene group less than desired for the group R in the product of formula (I).

The oxirane (III) may be prepared from a compound of the formula (II) by reacting with the methylide prepared from trimethyloxosulphonium iodide and sodium hydride. This reaction is generally achieved by adding powdered trimethyloxosulphonium iodide to a suspension of sodium hydride in dimethylsulphoxide and, after a few minutes, the ketone (II) is added, in equimolar amount. The reaction mixture may be warmed to accelerate the reaction and after several hours at 50 - 80°C the product is isolated in a conventional manner e.g. by pouring into water, solvent extraction and recrystallisation.

The reaction of the oxirane (III) with the Grignard reagent may be performed in a conventional manner as previously described for the ketone (II) but cuprous iodide may be added with advantage.

In the case of the alkenyl derivatives of formula (I) wherein R is an alkenyl group, hydrogenation can be used to obtain the corresponding saturated derivative wherein R is an alkyl group. The hydrogenation reaction is conducted in a conventional manner using a noble metal catalyst, e.g. platinum oxide, and is generally completed within 4 - 5 hours at room temperature. The product is isolated after removal of the catalyst by evaporation of the solvent and is further purified, if desired, as described above.

The starting materials of formula (II) are known compounds for example their preparation is described in British Patent Specification No. 1244530. The alkyl, cycloalkylalkyl and alkenyl halides are all readily accessible compounds from which the Grignard reagents may be prepared using standard procedures.

Since the compounds of the invention may contain one or more asymmetric centres, they will of course exist in various isomeric forms and the invention includes the individual isomers as well as the racemic mixtures.

Pharmaceutically acceptable acid addition salts of the compounds of the invention are those formed from acids which form nontoxic acid addition salts containing pharmaceutically-acceptable anions, such as the hydrochloride, hydrobromide, sulphate or bisulphate, phosphate or acid phosphate, acetate, maleate, fumarate, lactate, tartrate, citrate, gluconate, succinate and p-toluenesulphonate salts. The salts may be obtained by conventional procedures, e.g. by mixing solutions containing equimolar amounts of the free base and desired acid, and the required salt is collected by filtration, if insoluble, or by evaporation of the solvent.

The compounds of the invention and their pharmaceutically acceptable acid addition salts are anti-fungal agents, useful in combatting fungal infections in animals, including humans. For example they are useful in treating topical fungal infections in man caused by species of Candida, Trichophyton or Epidermophyton, or in mucosal infections caused by Candida albicans (e.g. thrush and vaginal candidiasis). They may also be used systemically in the treatment of systemic fungal infections caused by for example Candida albicans, Cryptococcus neoformans or Aspergillus fumigatus.

The in vitro evaluation of the anti-fungal activity of the compounds has been performed by determining the minimum inhibitory concentation (m.i.c.) of the test compounds in a suitable medium at which growth of the particular micro-organism fails to occur.

In practice a series of agar plates, each having the test compound incorporated at a particular concentration are inoculated with a standard culture of, for example, _Candida albicans_ and each plate is then incubated for 48 hours at 37°C. The plates are then examined for the presence or absence of growth of the fungus and the appropriate m.i.c. value is noted. Other micro-organisms used in such tests have included _Cryptococcus neoformans_, _Aspergillus fumigatus_, _Trichophyton rubrum_, _Epidermophyton floccosum_, _Coccidioides immitis_, and _Torulopsis_ glabrata.

The following results were obtained when the compounds of the Examples were evaluated _in vitro_ against _Candida albicans_. The m.i.c. recorded in the geometrical mean of the results obtained against a panel of 12 strains of the organism.

_In Vitro_ Activity vs. Candida Albicans

| Example | Mean m.i.c. | Example | Mean m.i.c. |
|---|---|---|---|
| 1 | 22.0 | 10 | 3.1 |
| 2 | 12.5 | 11 | 6.8 |
| 3a | 18.0 | 15 | 6.3 |
| 3b | 11.0 | | |
| 4 | 12.0 | 16 | 5.6 |
| 5 | 21.0 | 17 | 8.4 |
| 6 | 3.8 | 18 | 3.8 |
| 7 | 11.0 | 19 | 5.6 |
| 8 | 21.0 | 20 | 38.0 |
| 9 | 5.2 | 21 | 18.0 |

The *in vivo* evaluation of the compounds has also been carried out at a series of dose levels by intraperitoneal or intravenous injection or by oral administration, to mice which are inoculated with a strain of *Candida* albicans. Activity is based on the survival of a treated group of mice after the death of an untreated group of mice following 48 hours observation. The dose level at which the compound provides 50% protection against the lethal effect of the infection is noted.

For human use, the anti-fungal compounds of the invention can be administered alone, but will generally be administered in admixture with a pharmaceutical carrier selected with regard to the intended route of administration and standard pharmaceutical practice. For example, they may be administered orally in the form of tablets containing such excipients as starch or lactose, or in capsules either alone or in admixture with excipients, or in the form of elixirs or suspensions containing flavouring or colouring agents. They may be injected parenterally, for example, intravenously, intramuscularly or subcutaneously. For parenteral administration, they are best used in the form of a sterile aqueous solution which may contain other substances, for example, enough salts or glucose to make the solution isotonic.

For oral and parenteral administration to human patients, it is expected that the daily dosage level of the anti-fungal compounds of the invention will be comparable with that of anti-fungal agents currently in use, e.g. from 0.5 to 50 mg/kg (in divided doses) when administered by the parenteral routes, or from 2 to 200 mg/kg (in divided doses) when administered by the oral route.

Thus tablets or capsules of the compounds can be expected to contain from 30 mg to 1 g of active compound for administration singly or two or more at a time orally up to 4 times a day, while dosage units for parenteral administration will contain from 10 mg to 1 g of active compound. The physician in any event will determine the actual dosage which will be most suitable for an individual patient and it will vary with the age, weight and response of the particular patient. The above dosages are exemplary of the average case. There can, of course, be individual instances where higher or lower dosage ranges are merited, and such are within the scope of this invention.

Alternatively, the anti-fungal compounds of formula (I) may be administered in the form of a suppository or pessary or they may be applied topically in the form of a cream, ointment or dusting powder. For example, they may be incorporated into a cream consisting of an aqueous emulsion of polyethylene glycols or liquid paraffin; or they may be incorporated, at a concentration between 1 and 10%, into an ointment consisting of a white wax or white soft paraffin base together with such stabilizers and preservatives as may be required.

The following are examples of the preparation of the novel compounds of formula (I) according to the invention:

## EXAMPLE 1

2-(2,4-Dichlorophenyl)-1-(1-imidazolyl)-pent-4-en-2-ol

Magnesium turnings (4.0 g, 0.16 mole) were stirred under nitrogen for 1 hour. Dry diethyl ether (20 ml) was added and stirring continued while a solution of allyl bromide (10 g, 0.08 mole) in dry diethyl ether (200 ml) was added at such a rate as to maintain a gentle reflux. The mixture was heated under reflux for a further two hours and then cooled in an ice bath and a solution of 2',4'-dichloro-2-(1-imidazolyl)acetophenone (9 g, 0.035 mole) in a mixture of dry diethyl ether (150 ml) and dry tetrahydrofuran (30 ml) was added slowly with vigorous stirring. Further dry tetrahydrofuran (200ml) was added portionwise during the addition to maintain complete solution. The reaction mixture was stirred overnight at room temperature then treated with a solution of ammonium chloride (5 g) in water (50 ml). The organic phase was separated, dried over magnesium sulphate and the solvent evaporated to yield a yellow viscous oil which solidified on standing. Recrystallisation of the product from methanol - diethyl ether gave 2-(2,4-dichlorophenyl)-1-(1-imidazolyl)-pent-4-en-2-ol (8.9 g, 85%), m.p. 108 - 109°C. Found: C, 56.63; H, 4.76; N, 9.34. $C_{14}H_{14}Cl_2N_2O$ requires: C, 56.58; H, 4.75; N, 9.43%.

Alternatively the crude product was dissolved in diethyl ether and a solution of hydrogen chloride in diethyl ether added. The precipitate was collected and recrystallised from methanol-diethyl ether to give the hydrochloride salt as a colourless crystalline solid (9.2 g, 78%), m.p. 164 - 166°C.

EXAMPLE 2

2-(2,4-Dichlorophenyl)-1-(1-imidazolyl)-pentan-2-ol

A solution of 2-(2,4-dichlorophenyl)-1-(1-imidazolyl)-pent-4-en-2-ol (0.6 g) in methanol (20 ml) was hydrogenated at room temperature and 40 p.s.i. over platinum oxide catalyst (100 mg) until uptake of hydrogen was complete. The catalyst was removed by filtration and the solvent evaporated. The product was recrystallised from ethyl acetate to give 2-(2,4-dichlorophenyl)-1-(1-imidazolyl)-pentan-2-ol as colourless crystals (0.48 g, 80%), m.p. 154 - 157°C. Found: C, 55.7; H, 5.33; N, 9.34. $C_{14}H_{16}Cl_2N_2O$ requires: C, 56.2; H, 5.39; N, 9.36%.

EXAMPLE 3

2-(2,4-Dichlorophenyl)-1-(1-imidazolyl)-3-methyl-pent-4-en-2-ol

The Grignard reagent was prepared from crotyl bromide and treated with 2',4'-dichlorophenyl-2-(1-imidazolyl)acetophenone as described in Example 1. In this case the Grignard complex rearranged to yield 2-(2,4-dichlorophenyl)-1-(1-imidazolyl)-3-methyl-pent-4-en-2-ol as a mixture of diastereoisomers which were separated by chromatography on silica eluting with a mixture of hexane and isopropanol containing a trace of triethylamine. Fractions containing the first isomer to be eluted were combined and evaporated and the product recrystallised from a mixture of ethyl acetate and hexane to yield isomer (a) as colourless crystals (0.48 g, 39%) m.p. 147 - 149°C. Found: C, 57.65; H, 5.11; N, 8.97. $C_{15}H_{16}N_2Cl_2O$ requires: C, 57.89; H, 5.18; N, 9.00%.

Fractions containing the second isomer were combined and evaporated and the product recrystallised from hexane to yield isomer (b) as colourless crystals (0.42 g, 34%), m.p. 125 - 127°C. Found: C, 57.83; H, 5.12; N, 9.09. $C_{15}H_{16}N_2Cl_2O$ requires: C, 57.89; H, 5.18; N, 9.00%.

EXAMPLE 4

2-(2,4-Dichlorophenyl)-1-(1-imidazolyl)-3-methyl-pentan-2-ol

Reduction of 2-(2,4-dichlorophenyl)-1-(imidazolyl)-3-methyl-pent-4-en-2-ol (isomer b)(90 mg) by hydrogenation over platinum oxide as described in Example 2 gave 2-(2,4-dichlorophenyl)-1-(1-imidazolyl)-3-methyl-pentan-2-ol (72 mg, 80%), m.p. 212 - 215°C. Found: C, 56.5; H, 6.10; N, 8.58. $C_{15}H_{18}Cl_2N_2O$ requires: C, 57.5; H, 5.79; N, 8.94%.

EXAMPLE 5

2-(2,4-Dichlorophenyl)-1-(1-imidazolyl)-butan-2-ol

Magnesium turnings (0.49 g, 0.016 mole) was stirred under dry nitrogen for 1 hour. Dry diethyl ether (2 ml) was added and stirring continued while a solution of ethyl iodide (1.25 g, 0.008 mole) in dry diethyl ether (20 ml) was added at such a rate as to maintain a gentle reflux. The mixture was heated under reflux for a further hour and a solution of 2',4'-dichloro-2-(1-imidazolyl) acetophenone (1.02 g, 0.004 mole) in dry toluene (25 ml) was then added dropwise to the refluxing solution. The resulting grey suspension was heated under reflux for a further four hours with additions of dry toluene (10 ml portions) at hourly intervals. The reaction mixture was allowed to stand at room temperature overnight and a solution of ammonium chloride (2 g) in water (30 ml) added with stirring. The organic phase was separated, dried over magnesium sulphate and evaporated to yield a brown gum which was chromatographed on silica eluting with a mixture of hexane and isopropanol containing a trace of triethylamine. Recrystallisation of the product from cyclohexane gave 2-(2,4-dichlorophenyl)-1-(1-imidazolyl)-butan-2-ol (0.145 g, 13%) as colourless crystals, m.p. 104 - 106°C. Found: C, 54.81; H, 5.03; N, 9.45. $C_{13}H_{14}N_2Cl_2O$ requires: C, 54.76; H, 4.95; N, 9.82%.

EXAMPLES 6 -13

The following compounds were prepared following the procedure of Example 5 but starting with the appropriate alkyl halide. The product of Example 11 is a rearrangement product following reaction with the Grignard reagent prepared from cyclopropylmethyl bromide.

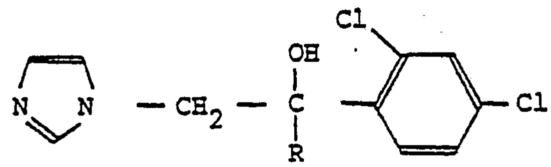

| Example No. | R | M.P. °C | Analysis % (Theoretical in brackets) C | H | N |
|---|---|---|---|---|---|
| 6 | $-(CH_2)_3CH_3$ | 121-124 | 57.2 (57.5 | 5.73 5.79 | 8.63 8.94) |
| 7 | $-C(CH_3)_3$ | 160-162 | 57.5 (57.5 | 5.53 5.79 | 8.68 8.94) |
| 8 | $-CH(CH_3)_2$ | 133-134 | 56.4 (56.2 | 5.47 5.39 | 9.52 9.36) |
| 9 | $-(CH_2)_4CH_3$ | 155-158 | 51.9 (51.8 | 5.30 5.32 | 6.21 6.71)* |
| 10 | $-(CH_2)_5CH_3$ | 147-148 | 53.1 (52.9 | 5.58 5.61 | 6.46 6.49)* |
| 11 | $-(CH_2)_2CH=CH_2$ | 120-125 | 57.6 (57.9 | 5.15 5.18 | 9.03 9.00) |
| 12 | $-(CH_2)_7CH_3$ | 92-93 | 61.7 (61.8 | 7.04 7.10 | 7.66 7.58) |
| 13 | $-(CH_2)_9CH_3$ | gum | ($^m/e$ = 396) | | |

* As oxalate salt

EXAMPLE 14

2-(2,4-Dichlorophenyl)-1-(1-imidazolyl)-octan-2-ol

A. Sodium hydride (50% dispersion in oil, 5.0 g, 0.11 mole) was washed with petrol to remove the oil, and dry dimethylsulphoxide (150 ml) added. The mixture was stirred under dry nitrogen at room temperature while finely ground trimethyloxosulphonium iodide (22.0 g, 0.1 mole) was added in portions over a period of 20 minutes. After a further 10 minutes a solution of 2',4'-dichloro-2-(1-imidazolyl)acetophenone (25.5 g, 0.1 mole) in dry dimethylsulphoxide (100 ml) was added over 20 minutes. The reaction mixture was heated at 65°C for 3 hours, cooled to room temperature and poured into a mixture of ice and water (1 l). The product was extracted with diethyl ether (3 x 500 ml) and the combined ether extracts were washed with water (2 x 250 ml), dried over $MgSO_4$ and evaporated to yield an oil. The oil was extracted with petroleum ether (b.p. 60 - 80°C, 5 x 250 ml) and the solvent evaporated. The resulting pale gum was crystallised from cyclohexane to give 2-(2,4-dichlorophenyl)-2-(1-imidazolylmethyl)oxirane (11.2 g, 42%) as colourless crystals, m.p. 85°C. Found (oxalate salt): C, 44.06; H, 3.52; N, 6.74. $C_{12}H_{10}Cl_2N_2O.1\frac{1}{2}.C_2H_2O_4$ requires: C, 44.57; H, 3.24; N, 6.93%.

B. Magnesium turnings (3.32 g, 0.14 mole) were stirred under dry nitrogen for 30 minutes. Dry tetrahydrofuran (50 ml) and a crystal of iodine were added and the stirring continued while a solution of 1-bromopentane (20.8 g, 0.14 mole) in dry tetrahydrofuran (300 ml) was added over 30 minutes maintaining a gentle exotherm.

The mixture was stirred for a further 30 minutes and then added to a suspension of cuprous iodide (2.63 g, 0.014 mole) in dry tetrahydrofuran (150 ml) at -30°C. The mixture was stirred at -30°C and a solution of 2-(2,4-dichlorophenyl)-2-(1-imidazolylmethyl)oxirane, (9.1 g, 0.035 mole) in dry tetrahydrofuran (150 ml) was added over 5 minutes. The temperature was maintained at -30°C for a further 10 minutes and the reaction mixture was then allowed to warm to room temperature and stirred overnight. A solution of ammonium chloride (40 g) in water (500 ml) was added and the mixture extracted with ethyl acetate (3 x 500 ml). The organic extracts were washed repeatedly with dilute ammonium hydroxide until the washings were colour free, with water (3 x 200 ml) and dried over $MgSO_4$. Evaporation of the solvent gave a gum which was chromatographed on silica eluting with a mixture of hexane and isopropanol. Fractions containing the product were combined and evaporated. The resulting gummy product was dissolved in diethyl ether and a solution of oxalic acid in diethyl ether added. The resulting oxalate salt was collected, and crystallised from acetone to give 2-(2,4-dichlorophenyl)-1-(1-imidazolyl)-octan-2-ol as colourless crystals (9.82 g, 66%) identical with the product of Example 10.

## EXAMPLE 15 - 19

The following compounds were prepared by the procedure of Example 14 but using the appropriate alkyl bromide in part B. Example 17 is a rearrangement product obtained by reaction with the Grignard prepared from cyclopropylmethyl bromide.

| Example No. | R | M.P. $^{o}C$ | Analysis % (Theoretical in brackets) C | H | N |
|---|---|---|---|---|---|
| 15 | 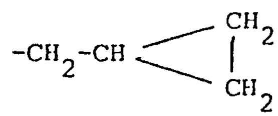 | 145-148 | 57.9 (57.9 | 5.11 5.15 | 9.09 9.02) |
| 16 | $-CH_2-CH(CH_3)_2$ | 147-149 | 56.8 (57.5 | 5.71 5.79 | 8.83 8.94) |
| 17 | $-(CH_2)_3CH=CH_2$ | 102-104 | 58.7 (59.2 | 5.46 5.54 | 8.90 8.62) |
| 18 | $-(CH_2)_2CH(CH_3)_2$ | 140-143 | 58.5 (58.7 | 6.15 6.16 | 8.35 8.56) |
| 19 | $-(CH_2)_6CH_3$ | glassy solid 92-95 | 60.8 (60.8 | 6.79 6.81 | 7.59 7.88) |

EXAMPLE 20

2-(4-Chlorophenyl)-1-(1-imidazolyl)-hexan-2-ol

The procedure of Example 5 was followed starting with 4'-chloro-2-(1-imidazolyl)acetophenone and reacting with the Grignard prepared from n-butyl-bromide to give 2-(4-chlorophenyl)-1-(1-imidazolyl)-hexan-2-ol, m.p. 145 - 148°C. Found: C, 64.71; H, 6.94; N, 9.77. $C_{15}H_{19}ClN_2O$ requires: C, 64.63; H, 6.87; N, 10.05%.

EXAMPLE 21

2-(2-Chlorophenyl)-1-(1-imidazolyl)-hexan-2-ol

The procedure of Example 14 was followed but starting with 2'-chloro-2-(1-imidazolyl)acetophenone and reacting with trimethyloxosulphonium iodine followed by reaction with the Grignard prepared from propylbromide to give 2-(2-chlorophenyl)-1-(1-imidazolyl)-hexan-2-ol, m.p. 103 - 104°C. Found: C, 64.6; H, 6.84; N, 10.05. $C_{15}H_{19}ClN_2O$ requires: C, 64.63; H, 6.87; N, 10.05%.

EXAMPLE 22

The following illustrate pharmaceutical compositions for the treatment of fungal infections:

(1) Capsule: 71 parts by weight of 2-(2,4-dichlorophenyl)-1-(1-imidazolyl)-hexan-2-ol are granulated with 3 parts maize starch and 22 parts lactose and then a further 3 parts of maize starch and 1 part magnesium stearate are added. The mixture is regranulated and filled into hard gelatin capsules.

(2) Cream: 2 parts by weight of 2-(2,4-dichlorophenyl)-1-(1-imidazolyl)-hexan-2-ol are dissolved in 10 parts of propylene glycol and mixed into 88 parts of a vanishing cream base.

(3) Pessary: 2 parts by weight of 2-(2,4-dichlorophenyl)-1-(1-imidazolyl)-hexan-2-ol are suspended in 98 parts of a warm liquified suppository base which is poured into moulds and allowed to solidify.

## CLAIMS

1. A compound of the formula:

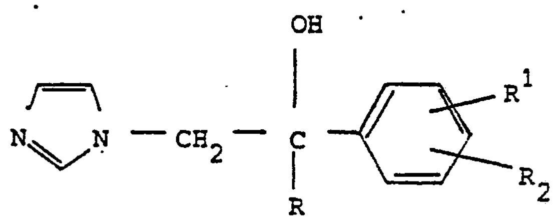

--- (I)

wherein R is a straight or branched chain alkyl or alkenyl group of from 2 to 10 carbon atoms, or a cycloalkyalkyl group of from 4 to 10 carbon atoms in which the cycloalkyl moiety contains 3 to 6 carbon atoms;

$R^1$ and $R^2$ are each independently a hydrogen or halogen atom, or a lower alkyl or lower alkoxy group;

and pharmaceutically acceptable salts thereof.

2. A compound as claimed in claim 1 wherein $R^1$ and $R^2$ are each chlorine in the 2 and 4 positions respectively.

3. A compound as claimed in claims 1 or 2 wherein R is a $C_3$-$C_6$ straight or branched chain alkyl group or a $C_3$-$C_4$ alkenyl group.

4. A compound as claimed in claim 3 wherein R is a propyl, n-butyl, iso-butyl, n-hexyl, or an allyl group.

5. 2-(2,4-Dichlorophenyl)-1-(1-imidazolyl)-pentan-2-ol,
2-(2,4-Dichlorophenyl)-1-(1-imidazolyl)-hexan-2-ol,
2-(2,4-Dichlorophenyl)-1-(1-imidazolyl)-4-methyl-pentan-2-ol,
2-(2,4-Dichlorophenyl)-1-(1-imidazolyl)-octan-2-ol,
2-(2,4-Dichlorophenyl)-1-(1-imidazolyl)-pent-4-en-2-ol.

6. A process for preparing a compound of the formula (I) as claimed in claim 1 which comprises reacting a compound of the formula:

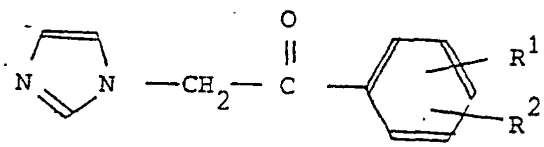

--- (II)

with a Grignard reagent of the formula RMgX where R, $R^1$ and $R^2$ are as defined in claim 1 and X is chloro bromo or iodo and optionally in the case where R is an alkenyl group, hydrogenating to give the corresponding alkyl group, and optionally forming a pharmaceutically acceptable acid addition salt of the product.

7. A process for preparing a compound of the formula (I) wherein R, $R^1$ and $R^2$ are as defined in claim 1 except that R may not be branched or unsaturated on the $\alpha$-carbon atom thereof, which comprises reacting a compound of the formula:

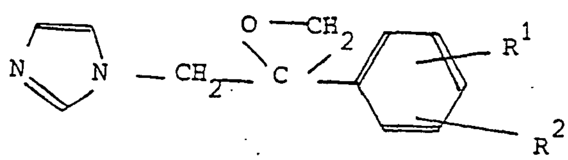

--- (III)

with a Grignard reagent of the formula $R^3MgX$ where $R^1$ and $R^2$ are as defined in claim 1 and $R^3$ is a straight or branched chain alkyl or alkenyl group of from 1 to 9 carbon atoms or a cycloalkylalkyl group of from 4 to 9 carbon atoms in which the cycloalkyl moiety contains 3 to 6 carbon atoms, and, optionally, in the case where $R^3$ is an alkenyl group, hydrogenating to give the corresponding alkyl group, and optionally forming a pharmaceutically acceptable acid addition salt of the product.

8. A process as claimed in claims 6 or 7 substantially as hereinbefore described with reference to the Examples.

9. A pharmaceutical composition comprising a compound of the formula (I) as claimed in any one of claims 1 to 5 or pharmaceutically acceptable salt thereof together with a pharmaceutically acceptable diluent or carrier.

10. A compound of the formula (I) or a pharmaceutically acceptable salt thereof as claimed in any one of claims 1 to 5 or a pharmaceutical composition as claimed in claim 9 for use in the treatment of fungal infections in animals including humans.

## CLAIMS FOR AUSTRIA

1. A process for preparing a compound of the formula:

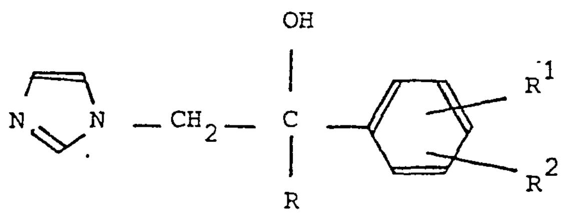

wherein R is a straight or branched chain alkyl or alkenyl group of from 2 to 10 carbon atoms, or a cycloalkylalkyl group of from 4 to 10 carbon atoms and $R^1$ and $R^2$ are each independently a hydrogen or halogen atom or a lower alkyl or lower alkoxy group; and pharmaceutically acceptable salts thereof which comprises reacting a compound of the formula:

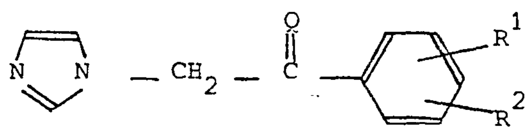

where Q is =O or 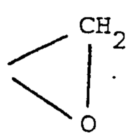 and $R^1$ and $R^2$ are as previously defined

with a Grignard reagent of the formula $R^3MgX$ where X is chloro, bromo or iodo and $R^3$ is as defined for R where Q is =O or as defined for R but one methylene group less when Q is 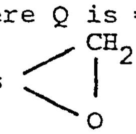, and optionally in the case where $R^3$ is an alkenyl group hydrogenating to give the corresponding alkyl group, and optionally forming a pharmaceutically acceptable acid addition salt of the product.

2. A process as claimed in claim 1 which comprises reacting a ketone of the formula:-

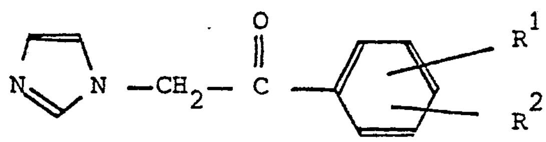

with a Grignard reagent of the formula RMgX, where R, $R^1$, $R^2$ and X are as defined in claim 1, and optionally in the case where R is an alkenyl group hydrogenating to give the corresponding alkyl group and optionally forming a pharmaceutically acceptable acid addition salt of the product.

3. A process as claimed in claim 1 which comprises reacting a compound of the formula:

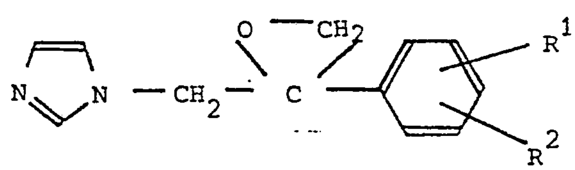

with a Grignard reagent of the formula $R^3MgX$ where $R^1$, $R^2$ and X are as defined in claim 1, and $R^3$ is as defined for R but one methylene group less, and optionally in the case where $R^3$ is an alkenyl group hydrogenating to give the corresponding alkyl group and optionally forming a pharmaceutically acceptable acid addition salt of the product.

4. A process as claimed in any one of claims 1 to 3 wherein $R^1$ and $R^2$ are each chlorine in the 2 and 4 positions respectively.

5. A process as claimed in any one of claims 1 to 4 wherein R is a $C_3$-$C_6$ straight or branched chain alkyl group or a $C_3$-$C_4$ alkenyl group.

6. A process as claimed in claim 5 wherein R is a propyl, n-butyl, iso-butyl, n-hexyl or an allyl group.

0023103

European Patent Office

# EUROPEAN SEARCH REPORT

Application number
EP 80 30 2237

| DOCUMENTS CONSIDERED TO BE RELEVANT | | | CLASSIFICATION OF THE APPLICATION (Int. Cl.³) |
|---|---|---|---|
| Category | Citation of document with indication, where appropriate, of relevant passages | Relevant to claim | C 07 D 233/60<br>C 07 D 405/06<br>A 61 K 31/415 |
| X | CHEMICAL ABSTRACTS, vol. 91, no. 7, 13th August 1979, page 709, column 2, no. 57005p<br>Columbus, Ohio, U.S.A.<br>& JP - A - 79 27563 (ROHM AND HAAS CO.) 01-03-1979<br>* Abstract *<br>-- | 1-5,9-10 | |
| D,X | GB - A - 1 244 530 (JANSSEN)<br>* Pages 1-3,5 *<br>---- | 1-5 | TECHNICAL FIELDS SEARCHED (Int. Cl.³)<br>C 07 D 233/60<br>A 61 K 31/415 |

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons
&: member of the same patent family, corresponding document

The present search report has been drawn up for all claims

| Place of search | Date of completion of the search | Examiner |
|---|---|---|
| The Hague | 01-10-1980 | DE BUYSER |

EPO Form 1503.1 06.78